(19) Europäisches Patentamt
European Patent Office
Office européen des brevets

(11) EP 0 863 864 B1

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(21) Anmeldenummer: **96938068.2**

(22) Anmeldetag: **04.11.1996**

(51) Int Cl.^7^: **C07C 49/21**, C07C 45/51, C11B 9/00, A61K 7/46

(86) Internationale Anmeldenummer:
**PCT/EP96/04797**

(87) Internationale Veröffentlichungsnummer:
**WO 97/17314 (15.05.1997 Gazette 1997/21)**

(54) **CARBONYLVERBINDUNGEN**

CARBONYL COMPOUNDS

COMPOSES CARBONYLE

(84) Benannte Vertragsstaaten:
**CH DE ES FR GB IT LI NL**

(30) Priorität: **10.11.1995 DE 19541963**

(43) Veröffentlichungstag der Anmeldung:
**16.09.1998 Patentblatt 1998/38**

(73) Patentinhaber: **Cognis Deutschland GmbH
40589 Düsseldorf (DE)**

(72) Erfinder:
- **MARKERT, Thomas
D-40789 Monheim (DE)**
- **SCHAPER, Ulf-Armin
D-47804 Krefeld (DE)**
- **PORRMANN, Volker
D-40723 Hilden (DE)**
- **FABER, Werner
D-47877 Willich (DE)**
- **TEN PIERIK, Theo
NL-5916 LE Venlo (NL)**

(56) Entgegenhaltungen:
**CH-A- 572 742** **DE-A- 1 643 176**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 863 864 B1

## Beschreibung

Die Erfindung betrifft spezielle Carbonylverbindungen der untenstehend angegebenen Struktur sowie deren Verwendung als Riechstoffe.

## Stand der Technik

Viele natürliche Riechstoffe stehen, gemessen am Bedarf, in völlig unzureichender Menge zur Verfügung. Beispielsweise sind zur Gewinnung von 1 kg Rosenöl 5.000 kg Rosenblüten notwendig; die Folgen sind eine sehr stark limitierte Weltjahresproduktion sowie ein hoher Preis. Es ist daher klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf an Geruchsverbesserern für Produkte des täglichen Bedarfs wie Kosmetika und Reinigungsmittel decken zu können.

Darüber hinaus besteht generell ein ständiger Bedarf an synthetischen Riechstoffen, die sich günstig und mit gleichbleibender Qualität herstellen lassen und erwünschte olfaktorische Eigenschaften haben, d.h. angenehme, möglichst naturnahe und qualitativ neuartige Geruchsprofile von ausreichender Intensität besitzen und in der Lage sind, den Duft von kosmetischen und Verbrauchsgütern vorteilhaft zu beeinflussen. Mit anderen Worten: Es besteht ein ständiger Bedarf an Verbindungen, die charakteristische neue Geruchsprofile bei gleichzeitig hoher Haftfestigkeit, Geruchsintensität und Strahlkraft aufweisen.

## Beschreibung der Erfindung

Es wurde gefunden, daß die Verbindungen der allgemeinen Formel (Ia) und/oder (Ib) die oben genannten Forderungen in jeder Hinsicht ausgezeichnet erfüllen und in vorteilhafter Weise als Riechstoffe mit unterschiedlich nuancierten Geruchsnoten mit guter Haftfestigkeit eingesetzt werden können.

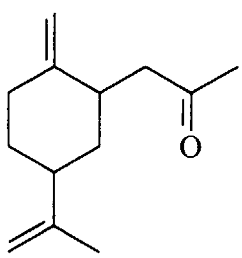

(Ia)

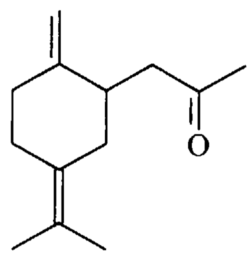

(Ib)

Gegenstand der vorliegenden Erfindungen sind daher zunächst die Carbonylverbindungen (Ia) und/oder (Ib).

Ein weiterer Gegenstand der vorliegenden Erfindung ist die Verwendung der Carbonylverbindungen (Ia) und/oder (Ib) als Riechstoffe.

Die erfindungsgemäßen Carbonylverbindungen zeichnen sich durch eine Geruchscharakteristik, in der fruchtige, grüne sowie an Ionon erinnernde Noten dominieren, aus. Sie weisen eine ausgezeichnete Stabilität in Rezepturen der Kosmetik und Gebrauchsparfümerie auf.

Die Herstellung der Verbindungen (I) erfolgt nach an sich bekannten Syntheseverfahren der organischen Chemie. Vorzugsweise werden die Verbindungen (I) mittels der sogenannten Carroll-Reaktion aus den entsprechenden Allylalkoholen hergestellt. Bei diesen handelt es sich um Perillaalkohol und/oder Isoperillaalkohol.

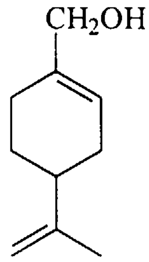

Perilla-Alkohol

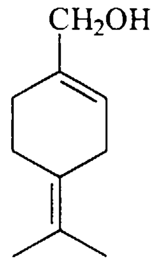

Isoperilla-Alkohol

Unter **Carroll-Reaktion** wird dabei im Rahmen der vorliegenden Erfindung die Überführung von Allylalkoholen in gamma-delta-ungesättigte Ketone verstanden. Der Allylalkohol wird dabei in einer ersten Variante der Reaktion durch Umsetzung mit Acetessigester in den entsprechenden Acetessigsäureallylester überführt, aus dem dann durch [3,3]-sigmatrope Umlagerung (Claisen-Umlagerung) die alpha-Allylacetessigsäure entsteht, die nach einer thermischen Decarboxylierung das gewünschte gamma-delta-ungesättigte Keton (I) ergibt. Der Acetessigsäureallylester kann dabei in Substanz eingesetzt oder in situ gebildet werden.

In einer zweiten Variante der Carroll-Reaktion, die beispielsweise in einem Übersichtsartikel von G.B. Bennett angesprochen ist (vergleiche: Synthesis 1977, Seiten 589-606), setzt man den Allylalkohol mit einem Vinylether oder einem Alkoxyalken um. Beim Einsatz von Vinylethern entstehen dabei Aldehyde, beim Einsatz von Alkoxyalkenen Aldehyde oder Ketone, je nach der Natur des verwendeten Alkoxyalkens. Beispielsweise ergibt die Umsetzung von Zimtalkohol mit 1-Methoxypropen einen Aldehyd, die Umsetzung von Zimtalkohol mit 2-Methoxypropen ein Keton. Als intermediäre Verbindungen bei diesen Reaktionen kann man Acetale beziehungsweise Allylvinylether annehmen.

In einer bevorzugten Ausführungsform der Erfindung setzt man in der Carroll-Reaktion Perilla- und/oder Isoperillaalkohol mit 2-Methoxypropen um. Der intermediäre Allylvinylether kann dabei isoliert oder direkt weiter in situ der anschließenden [3,3]-sigmatropen Umlagerung zum entsprechenden Keton (I) unterworfen werden.

Im untenstehenden Schema 1 ist der Verlauf der Carroll-Reaktion beispielhaft für die Umsetzung von Perillaalkohol mit 2-Methoxypropen skizziert (für die experimentelle Durchführung der Reaktion sei auf das untenstehende Beispiel 2 verwiesen).

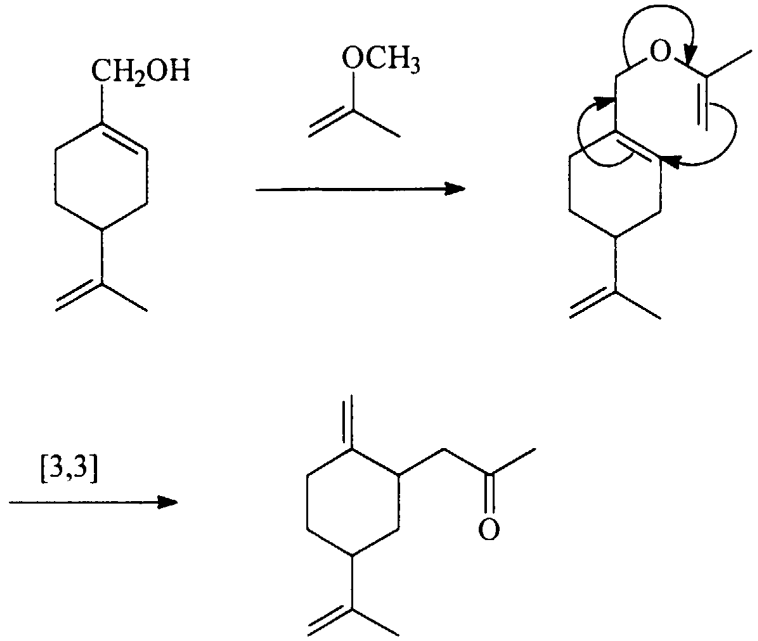


Schema 1

Ein weiterer Gegenstand der Erfindung ist daher ein Verfahren zur Herstellung der Carbonylverbindungen (I) durch Carroll-Reaktion aus Perilla- und/oder Isoperillaalkohol. Dabei ist die Variante, bei der im Zuge der Carroll-Reaktion die [3,3]-sigmatrope Umlagerung über den Allylvinylether erfolgt und bei der der zu Beginn der Synthese eingesetzte Allylalkohol mit einem 2-Methoxypropen umgesetzt wird, bevorzugt, da bei dieser Art der Herstellung eine besonders hohe Produktreinheit sichergestellt ist und somit die erwünschte hohe geruchliche Qualität der Verbindungen (I) sichergestellt ist.

Die Verbindungen (I) bedeutet, zeichnen sich durch einen fruchtig-grünen Geruch aus, der durch Iononaspekte an Intensität gewinnt.

In Parfüm-Kompositionen verstärken die Verbindungen (I) die Harmonie und Ausstrahlung und Natürlichkeit sowie auch die Haftung, wobei die Dosierung unter Berücksichtigung der übrigen Bestandteile der Komposition auf die jeweils angestrebte Duftnote abgestimmt wird.

Daß die Carbonylverbindungen (I) fruchtig-grüne Noten aufweisen, war nicht vorhersehbar und ist damit eine weitere Bestätigung für die allgemeine Erfahrung, daß die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluß der chemischen Struktur auf die Duftwahrnehmung hinreichend erforscht sind, somit also normalerweise nicht vorhergesehen werden kann, ob ein geänderter Aufbau bekannter Riechstoffe überhaupt zur Änderung der olfaktorischen Eigenschaften führt und ob diese Änderungen positiv oder negativ beurteilt werden.

Die Verbindungen der Formel (I) eignen sich aufgrund ihres Geruchsprofils insbesondere auch zur Modifizierung und Verstärkung bekannter Kompositionen. Hervorgehoben werden soll insbesondere ihre außerordentliche Geruchsstärke, die ganz allgemein zur Veredelung der Komposition beiträgt.

Die Verbindungen der Formel (I) lassen sich mit zahlreichen bekannten Riechstoffingredientien, z.B. anderen Riechstoffen natürlichen, synthetischen oder partialsynthetischen Ursprungs, etherischen Ölen und Pflanzenextrakten kombinieren. Die Palette der natürlichen Riechstoffe kann dabei sowohl leicht- als auch mittel- und schwerflüchtige Komponenten und diejenige der synthetischen Riechstoffe Vertreter aus praktisch allen Stoffklassen umfassen. Beispiele sind:

(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl,Myrrheöl, Olibanumöl

(b) Alkohole wie Farnesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],

(c) Aldehyde wie Citral, Helional[R], α-Hexylzimtaldehyd, Hydroxycitronellal, Lilial[R] [p-tert.-Butyl-α-methyldihydrozimtaldehyd], Methylnonylacetaldehyd,

(d) Ketone wie Allylionon, α-Ionon, β-Ionon, Isoraldein, Methylionon,

(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat,

(f) Lactone wie gamma-Undecalacton, 1-Oxaspiro[4.4]nonan-2-on,

sowie verschiedene weitere in der Parfümerie oft benutzte Komponenten wie Moschus- und Sandelholz-Riechstoffe, Indol, p-Menthan-8-thiol-3-on, Methyleugenol, Ambroxan.

Bemerkenswert ist ferner die Art und Weise, wie die Verbindungen der Struktur (I) die Geruchsnoten einer breiten Palette bekannter Kompositionen abrunden und harmonisieren, ohne aber in unangenehmer Weise zu dominieren. 4-Phenyl-hexan-2-on ist in dieser Hinsicht ganz besonders hervorzuheben.

Die erfindungsgemäßen Verbindungen (I) enthalten Chiralitätszentren, so daß diese Verbindungen in verschiedenen Raumformen existieren können. Im Rahmen üblicher Synthesen fallen die erfindungsgemäßen Verbindungen als Gemische der entsprechenden Isomeren an und werden als solche als Riechstoffe verwendet.

Die einsetzbaren Anteile der erfindungsgemäßen Verbindungen oder deren Gemische in Riechstoffkompositionen bewegen sich von 1 bis 70 Gewichtsprozent, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Puder, Aerosole, Zahnpasten, Mundwässer, Deodorantien als auch in der alkoholischen Parfümerie (z.B. Eaux de Cologne, Eaux de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,05 bis 2 Gewichtsprozent, bezogen auf das gesamte Produkt, zugesetzt. Diese Werte sollen jedoch keine Grenzwerte darstellen, da der erfahrene Parfümeur auch mit noch geringeren Konzentrationen Effekte erzielen oder aber mit noch höheren Dosierungen neuartige Komplexe aufbauen kann.

Die folgenden Beispiele sollen den Gegenstand der Erfindung erläutern und sind nicht einschränkend aufzufassen.

## Beispiele

### Nomenklatur

Perillaalkohol = (4-Isopropenyl-cyclohex-1-en-1-yl)-methanol
Isoperillaalkohol = (4-Isopropyliden-cyclohex-1-enyl)-methanol

### Beispiel 1:

Umsetzung von β-Pinenoxid zu einem Gemisch aus Perillaalkohol und Isoperillaalkohol

Ansatz:
1) 102 g β-Pinenoxid (0,67 mol) technischer Qualität (92%-ig; Fa. Acros)
2) 500 ml entmineralisiertes Wasser
3) 200 ml Trockeneis.

Ausführung: In einem 2 l Becherglas wurden 102 g β-Pinenoxid in 500 ml Wasser emulgiert und portionsweise Trockeneis eingetragen. Die exotherme Reaktion wurde 6 Stunden weitergeführt bis die Reaktionskontrolle über GLC keine weitere Umsetzung mehr anzeigte. Dann wurde das Reaktionsprodukt mit Ether aus der wäßrigen Phase extrahiert, die vereinigten organischen Phasen wurden mit Wasser gewaschen, über Magnesiumsulfat getrocknet und am Rotationsverdampfer von Lösungsmittel befreit. 90,4 g Rohprodukt wurden nach Zusatz von 0,45 g para-Pyridiniumtosylat in eine Kugelrohrdestille überführt und dort im Hochvakuum bei Ofentemperaturen zwischen 140 und 175 °C langsam überdestilliert.

Es wurden 65,1 g Rohprodukt erhalten, die durch fraktionierte Destillation an einer Drehband-Kolonne weiter aufgereinigt wurden. Eine Auftrennung in Perillaalkohol und Isoperillaalkohol war an der Drehbandkolonne ebenfalls möglich. Die NMR- und IR-Spektren entsprachen den literaturbekannten Daten.

**Beispiel 2:**

Umsetzung eines Gemischs aus Isoperillaalkohol und Perillaalkohol zu 1-(5-Isopropyliden-2-methylen-cyclohexyl)-2-propanon und zu 1-(5-(2'-Prop-2'-enyl)-2-methylencyclohexyl)-2-propanon.

Ansatz: 4) 75 g (0,49 mol) Perilla-/Isoperillaalkohol-Gemisch aus Beispiel 1
5) 42 g (0,57 mol) Isopropenylmethylether (92 %ig, Fa. Acros)
6) 150 g Cyclohexan (99 %ig)

Ausführung: In einem 500 ml Stahlautoklaveneinsatz wurden die Komponenten 4) bis 6) vorgelegt und bei Raumtemperatur mit 10 bar Stickstoff-Überdruck inertisiert. Nach einer Reaktionszeit von 7 Stunden bei 190 °C und 30 bar war gaschromatographisch kein Edukt mehr detektierbar, so daß abgebrochen wurde. Der Ansatz wurde am Rotationsverdampfer von Lösungsmittel befreit. 95,6 g Rohprodukt wurden durch Kugelrohrdestillation vorgereinigt und an einer Drehbandkolonne fraktioniert. Der Hauptlauf von 51,8 g enthielt ein Gemisch der gewünschten Isomeren in 95 %iger Reinheit. Siedepunkt des Gemischs: 57-58 °C / 0,03 mbar.

Geruchsbeschreibung: fruchtig, grün, Ionon

**Kompositionsbeispiel:** DPG = Dipropylenglykol

| Gewichtsteile | | |
|---|---|---|
| 1 | Ambroxan | (Fa. Henkel) |
| 3 | Oxyphenylon | |
| 5 | Allylamylglykolat | |
| 5 | Eugenol 10%ig in DPG | |
| 5 | Damascenone 10%ig in DPG | (Fa. Firmenich) |
| 7 | Damascone Beta 10%ig in DPG | (Fa. Firmenich) |
| 9 | Aldehyd C14 sog. | |
| 15 | Vanillin | |
| 20 | Bergamotteöl Berg.-Frei | |
| 20 | Floramat | (Fa. Henkel) |
| 25 | Heliotropin | |
| 25 | Dimethylbenzylcarbinylacetat | |
| 40 | Cyclohexylsalicylat | (Fa. Henkel) |
| 45 | Troenan | (Fa. Henkel) |
| 50 | Sandelice | (Fa. Henkel) |
| 70 | Hedione | (Fa. Firmenich) |
| 140 | Iso E Super | (Fa. IFF) |
| 190 | Isoraldein 70 | (Fa. Givandan-Roure) |
| 275 | Galaxolide 50 DEP | (Fa. IFF) |
| 50 | **Dipropylenglykol** | |
| 1000 | | |

Durch Ersetzen der 50 Gewichtsteile Dipropylenglykol durch die Substanz gemäß Beispiel 2 wurde die Himbeernote der Grundkomposition unterstützt, abgerundet und deutlich verstärkt.

**Patentansprüche**

**1.** Carbonylverbindungen der Formel (Ia)

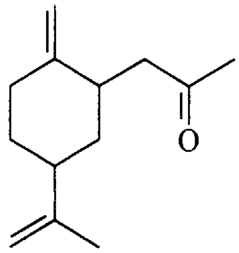

(Ia)

**2.** Carbonylverbindungen der Formel (Ib)

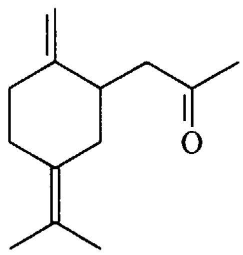

(Ib)

**3.** Gemisch der Carbonylverbindungen der Formeln (Ia) und (Ib)

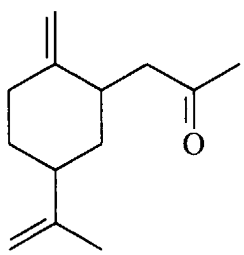

(Ia)

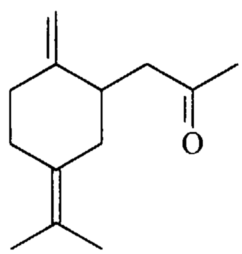

(Ib)

**4.** Verwendung der Carbonylverbindungen der Formel (Ia)

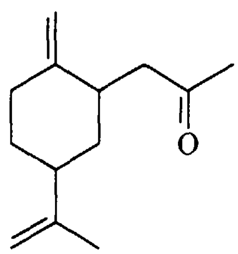

(Ia)

als Riechstoffe.

**5.** Verwendung der Carbonylverbindungen der Formel (Ib)

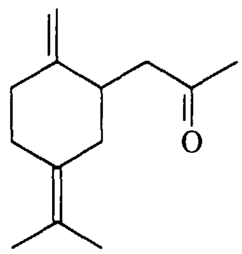

(Ib)

als Riechstoffe.

**6.** Verwendung von Gemischen der Carbonylverbindungen der Formeln (Ia) und (Ib)

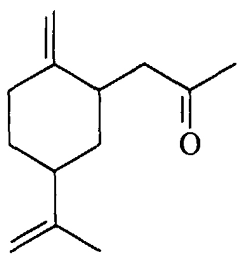

(Ia)

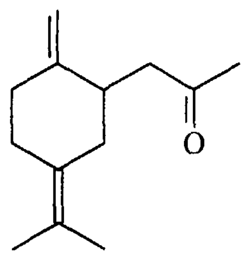

(Ib)

als Riechstoffe.

**7.** Riechstoffkompositionen mit einem Gehalt an einem oder mehreren Carbonylverbindungen (Ia) und/oder (Ib) in einer Menge von 1 - 70 Gew.-% (bezogen auf die gesamte Komposition).

**8.** Verfahren zur Herstellung von Carbonylverbindungen der allgemeinen Formel Ia und/oder Ib

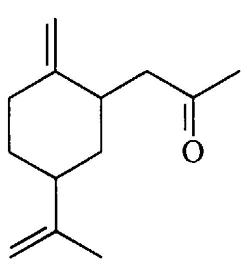

(Ia)

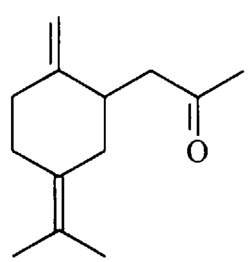

(Ib)

durch Carroll-Reaktion aus Perilla- und/oder Isoperillaalkohol und 2-Methoxypropen.

## Claims

1. Carbonyl compounds corresponding to formula (Ia):

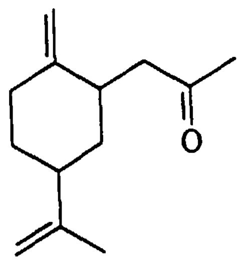

(Ia)

2. Carbonyl compounds corresponding to formula (Ib):

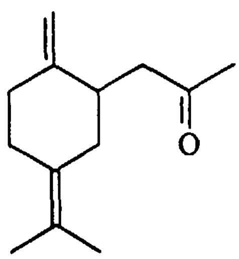

(Ib)

3. A mixture of carbonyl compounds corresponding to formulae (Ia) and (Ib):

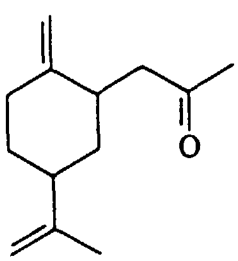

(Ia)

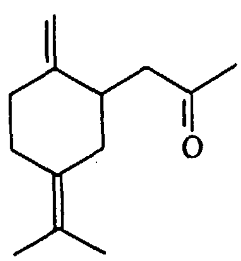

(Ib)

4. The use of the carbonyl compounds corresponding to formula (Ia):

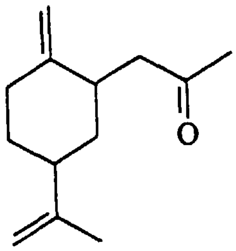

(Ia)

as perfumes.

5. The use of the carbonyl compounds corresponding to formula (Ib):

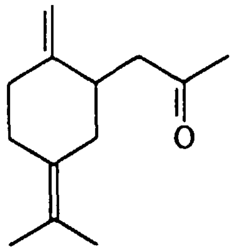

(Ib)

as perfumes.

6. The use of mixtures of carbonyl compounds corresponding to formulae (Ia) and (Ib):

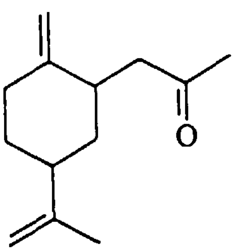

(Ia)

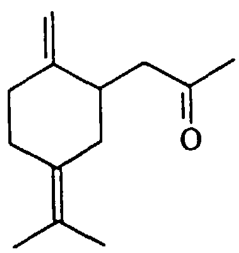

(Ib)

as perfumes.

7. Perfume compositions containing one or more carbonyl compounds (Ia) and/or (Ib) in a quantity of 1 to 70% by weight (based on the composition as a whole).

8. A process for the production of carbonyl compounds corresponding to general formula (Ia) and/or (Ib):

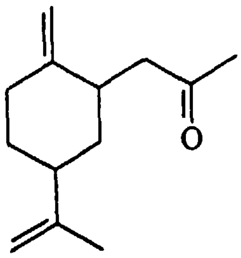
(Ia)

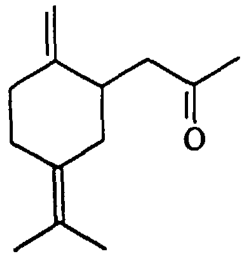
(Ib)

by Carroll reaction from perilla and/or isoperilla alcohol and 2-methoxypropene.

## Revendications

**1.** Composés carbonyle de formule (Ia)

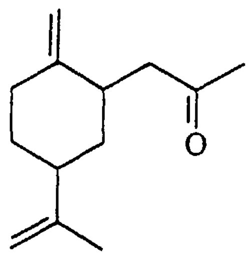
(Ia)

**2.** Composés carbonyle de formule (Ib)

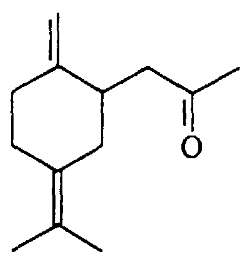
(Ib)

**3.** Mélange des composés carbonyles de formules (Ia) et (Ib)

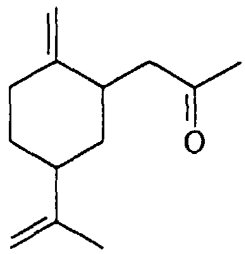

(Ia)

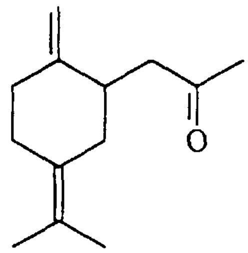

(Ib)

**4.** Utilisation des composés carbonyle de formule (Ia)

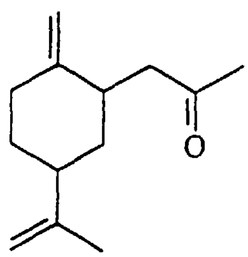

(Ia)

comme parfums.

**5.** Utilisation des composés carbonyle de formule (Ib)

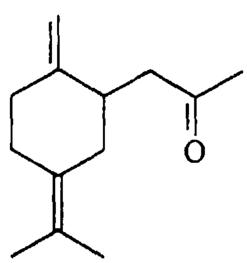

(Ib)

comme parfums.

**6.** Utilisation des mélanges de composés carbonyle des mélanges (Ia) et (Ib)

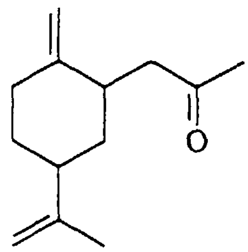

(Ia)

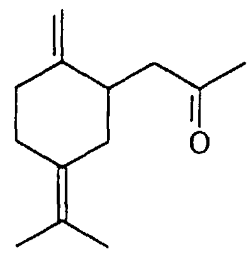

(Ib)

comme parfums.

**7.** Compositions de parfum contenant un ou plusieurs composés de carbonyle (la) et/ (Ib) en une quantité de 1 à 70 % en poids (par rapport à l'ensemble de la composition).

**8.** Procédé de production de composés carbonyle de formule générale (la) et/ou (Ib)

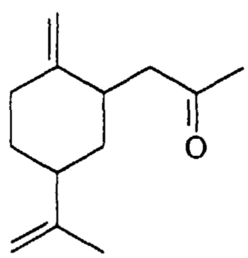

(Ia)

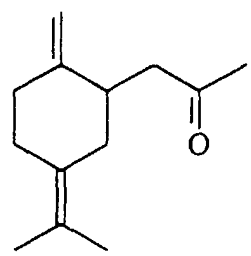

(Ib)

par une réaction de Carroll à partir d'alcool de périlla et/ou d'alcool isopérillique et de 2-méthoxypropène.